(19)

Europäisches
Patentamt
European
Patent Office
Office européen
des brevets

(11) **EP 2 343 075 A1**

(12) **EUROPEAN PATENT APPLICATION**

(43) Date of publication:
   **13.07.2011 Bulletin 2011/28**

(51) Int Cl.:
   ***A61K 31/549*** *(2006.01)*    ***A61P 25/00*** *(2006.01)*

(21) Application number: **10150027.0**

(22) Date of filing: **04.01.2010**

(84) Designated Contracting States:
   **AT BE BG CH CY CZ DE DK EE ES FI FR GB GR HR HU IE IS IT LI LT LU LV MC MK MT NL NO PL PT RO SE SI SK SM TR**
   Designated Extension States:
   **AL BA RS**

(71) Applicant: **Neurotec Pharma, S.L.**
   **08028 Barcelona (ES)**

(72) Inventors:
   • **Pugliese, Marco**
     **E-08810, Sant Pere de Ribes - Barcelona (ES)**
   • **Espinosa Parrilla, Juan Francisco**
     **E-08915, Badalona - Barcelona (ES)**

   • **Virgili Treserres, Noemí**
     **E-43883, Roda de Barà - Tarragona (ES)**
   • **Mancera Aroca, Pilar**
     **E-43883, Roda de Barà - Tarragona (ES)**
   • **Pastén Zamorano, Andrea**
     **E-08026, Barcelona (ES)**
   • **Mahy-Gehenne, Josette-Nicole**
     **E-07460, Pollença - Mallorca (ES)**
   • **Rodríguez Allué, Manuel**
     **E-08980, Sant Feliu de Lobregat - Barcel (ES)**

(74) Representative: **ABG Patentes, S.L.**
   **Avenida de Burgos 16D**
   **Edificio Euromor**
   **28036 Madrid (ES)**

(54) **Diazoxide for use in the treatment a central nervous system (CNS) autoimmune demyelinating disease**

(57)    The invention relates to the use of diazoxide or a pharmaceutically acceptable salt thereof at low doses to treat a CNS autoimmune demyelinating disease selected from selected from multiple sclerosis (MS), acute disseminated encephalomyelitis (ADEM), and post-vaccinal encephalitis (PVE).

EP 2 343 075 A1

**Description**

**FIELD OF THE INVENTION**

The invention relates to the use of diazoxide or a pharmaceutically acceptable salt thereof at low doses to treat a central nervous system (CNS) autoinmune demyelinating disease selected from multiple sclerosis (MS), acute disseminated encephalomyelitis (ADEM), post-vaccinal encephalitis (PVE) and to compositions comprising low doses of diazoxide for use in the treatment of a mammal afflicted with a CNS autoimmune demyelinating disease.

**BACKGROUND OF THE INVENTION**

Multiple Sclerosis (MS) is an autoimmune, chronic inflammatory and demyelinating central nervous system (CNS) disease that occurs in genetically susceptible individuals and involving immunological factors such as antibodies, complement and mediators of the innate immune response.

MS is classified as idiopathic inflammatory demyelinating disease, and is one of the most common causes of neurological disability in young adults after trauma and epilepsy (Noseworthy JH et al. Multiple sclerosis. N Engl J Med 2000, 343:938-52).

MS is characterized by recurrent attacks presenting multifocal neurologic signs and symptoms and with varying degrees of recovery. Clinical manifestations include visual loss, eye movement disorders, sensory symptoms, weakness, spasticity, ataxia, bladder dysfunction, and cognitive, affective and paroxysmal disorders. The pathological hallmark of MS is the presence of focal areas of inflammatory-mediated demyelination of the brain and spinal cord white matter.

MS is a disease of high latitudes (areas and countries above 40 degrees latitude) and the Western hemisphere. Low prevalence was considered less than 5 cases per 100,000 persons, an intermediate prevalence was 5 to 30 per 100,000 persons, and a high prevalence was more than 30 per 100,000 persons (Kurtzke JF. Multiple sclerosis: changing time. Neuroepidemiology 1991, 10:1-8). The prevalence is highest in the northern Europe, southern Australia and the middle part of North America, whereas is less present in Japan and China. For unexplained reasons, MS affects twice as many women as it does men and the disease usually manifests between 20 and 40 years. Fifteen years after onset, 50% of patients are unable to walk. Life expectancy is at least 25 years from disease onset with most patients dying from unrelated causes.

MS is primarily an inflammatory disorder of the brain and spinal cord in which focal lymphocytic infiltration leads to damage of the myelin and axons. Initially, inflammation is transient and remyelination occurs but is not durable. However, over time the pathological changes become dominated by widespread microglial activation associated with extensive and chronic neurodegeneration, the clinical correlate of which is progressive accumulation of disability (Compston A and Coles A. Multiple Sclerosis. Lancet 2008, 372:1502-17).

The majority of studies on the pathogenesis of MS have been performed in the animal model experimental autoimmune encephalomyelitis (EAE), which can be induced by injecting susceptible rodent strains with myelin proteins or peptides. These animals subsequently develop either a monophasic inflammatory CNS disease or various forms of chronic or relapsing-remitting EAE and, in their CNS lesions, CD4+ T cells, monocytes/activated microglia and other cell populations and humoral factors are found (Martin R et al. Immunological aspects of demyelinating diseases. Ann Rev Immunol 1992, 10:153-187).

Microglial cells are crucial in the development of CNS inflammation. This cell population is ubiquitously distributed in non-overlapping territories throughout the CNS and represents a significant fraction of adult CNS cells (e.g. 5-20%). In functional terms, microglia represents the network of immune accessory cells throughout the brain, spinal cord and eye structures functioning as an intrinsic sensor of threats. The high sensitivity of microglial cells to the CNS microenvironment changes enables them to function as sentinels (Kreutzberg GW. Microglia: a sensor for pathological events in the CNS. Trends Neurosci 1996, 19:312-3188). In MS, these cells might sustain and propagate inflammation within the CNS during autoimmune inflammation through antigen presentation and/or cytokine/chemokine secretion leading to gliosis and axonal damage (Heppner FL et al. Experimental autoimmune encephalomyelitis repressed by microglial paralysis. Nat Med 2005, 11:146-152).

Microglia and macrophages are activated in response to neuronal damage and they may mediate the inflammatory response by producing proinflammatory cytokines, generating reactive oxygen or nitrogen species, producing excitatory aminoacids, activating complement components, or releasing proteolytic and lipolytic enzymes (Lucchinetti CF et al. Multiple sclerosis: lessons from neuropathology. Semin Neurol. 1998, 18:337-349).

There is no cure for MS. At present, there is no treatment that can influence significantly the course of the disease, but only palliative therapies that aim to improve symptoms, although they can't be used for all patients, are not extremely effective and with important undesirable effects. At present, six treatments are currently approved by Food and Drug Administration and European Medicines Agency (EMEA): three beta interferons (IFNs) (Goodin DS. Interferon-beta-therapy in multiple sclerosis: evidence for a clinically relevant dose response. Drugs 2001, 61:1693-1703), glatiramer acetate (GA), formerly known as copolymer-1 (Neuhaus O et al. Mechanisms of action of glatiramer acetate in multiple sclerosis. Neurology 2001, 56:702-708), natalizumab (Yednock TA et al. Prevention of experimental autoimmune encephalomyelitis by antibodies against $\alpha 4\beta 1$ integrin. Nature 1992, 356:63-66) and mitoxantrone (Goodin DS et al. The use of mitoxantrone (Novantrone) for the treatment of multiple sclerosis. Report of the Therapeutics and Technology Assessment subcommittee of the American Academy of Neurology. Neurology 2003, 61:1332-1338).

Delivery of IFNs or GA require frequent intramuscular or subcutaneous injections, which can produce many side effects including flu-like symptoms, dermal reactions, anxiety palpitations, dyspnea and, in case of IFNs, neutralizing antibodies that achieve less therapeutic effects (Francis GS et al. Interferon-beta1-a in MS: results following development of neutralizing antibodies in PRISMS. Neurology 2005, 65:48-55). In addition, some reports include the development or worsening of preexisting thyroid dysfunction, myasthenia gravis, systemic lupus erythematosus, rheumatoid arthritis and other inflammatory arthritis, urticaria, psoriasis and a severe capillary leak syndrome in patients with a coexisting monoclonal gammopathy. Mitoxantrone should be administered only by experienced personnel trained in the administration of chemotherapeutic agents. Extravasation causes permanent staining of the subcutaneous tissues and gastrointestinal symptoms (nausea and vomiting) are rarely severe. Hair loss is rarely but menstrual dysfunction (irregularity or premature menopause) occurs in up to 25% of premenopausal patients (Hartung HP et al. Mitoxantrone in progressive multiple sclerosis: a placebo-controlled, double-blind, randomised multicentre trial. Lancet 2002, 360:2018-2025). Finally, despite natalizumab trials showed significant benefits of treatment on both clinical and magnetic resonance imaging (MRI) measures of disease, these therapeutic benefits seemed to be conferred with few notable side effects of therapy, especially with developing progressive multifocal leukoencephalopathy (PML) with death of patients (Rudick RA et al. Natalizumab plus interferon beta-1a for relapsing multiple sclerosis. N Engl J Med 2006, 354:911-923).

In summary, it is highly desirable to provide new therapeutic agents for the treatment of MS applicable to all clinical presentations of the disease, easily to administer and with no side effects in their acute and long-term therapeutic use.

It has been shown that activated microglia, one of the elements involved in MS inflammation, expresses $K_{ATP}$ channels (Ramonet D et al. Putative glucosensing property in rat and human activated microglia. Neurobiol Dis 2004, 17:1-9) and it has been suggested that this may represent a new therapeutic target.

WO2006/000607 Al discloses the potential use of $K_{ATP}$ channel openers (KCO) for the treatment of CNS chronic inflammation associated to a disease in mammals. The application also disclosed that suitable doses were to be determined by a doctor or veterinary but would lie within the range of 0.01 to 1000 mg/kg/day.

Diazoxide is a benzothiadiazine that acts as a $K_{ATP}$ channel opener (Mannhold R. KATP channel openers: structure-activity relationships and therapeutic potential. Med Res Rev 2004, 24:213-66) and that has been used until now against human hypertension and hypoglycemia. Diazoxide is administered parenterally in the form of minibolus of 1-3 mg/kg (37-111 mg/m$^2$) up to a maximum of 150 mg (185 mg/m$^2$) in a single injection every 5 to 15 minutes to treat hypertensive emergencies (Hyperstat®) and it is also used orally to manage refractory malignant hypertension in a dose range of 600-800 mg per day (22,200-29,600 mg/m$^2$) (Fang P, MacDonald I, Laver M, Hua A, Kincaid-Smith P. Oral diazoxide in uncontrolled malignant hypertension. Med J Aust. 1974 Oct 26;2(17):621-4). Effective oral doses in adult humans for the treatment of hypoglycemia are 3-8 mg/kg/day (111-296 mg/m$^2$) (Proglycem®). However, these doses frequently cause severe side effects such as oedema and hirsutism.

Until now no studies have investigated the effectiveness of diazoxide in MS. In addition, research related to the dose-response effect of diazoxide in different brain damage situations has shown that high doses are required to get some beneficial effect. Gantenbein et al. showed that 100 mg/kg (300 mg/m$^2$) of diazoxide are required to reduce the bupivacaine-induced acute CNS toxicity in mice by increasing the period of latency of the convulsions caused by bupivacaine (Gantenbein M et al. 1995. Life Sciences, 57: 113-116). Farkas E et al. showed that 5 mg (143 mg/m$^2$) diazoxide in rats and dimethyl sulphoxide prevents cerebral hypoperfusion-related learning dysfunction and brain damage after carotid artery occlusion (Farkas E et al. Brain Research. 2004, 1008:252-260). Lenzser G et al. showed that diazoxide preconditioning is capable of attenuating global cerebral ischemia-induced blood-brain barrier permeability when administered to rats at 20 mg/kg (120 mg/m$^2$) but shows no effect at 6 mg/kg (36 mg/m$^2$) (Lenzser G et al. Brain Research. 2005, 1051:72-80).

Notwithstanding the above, it would be of great interest to develop a method of treatment of demyelinating diseases such as multiple sclerosis (MS), acute disseminated encephalomyelitis (ADEM) or post-vaccinal encephalitis (PVE) which is devoid of undesirable side effects associated with the use of KCOs such as hyperglycemia.

## SUMMARY OF THE INVENTION

Inventors have surprisingly found that daily doses of diazoxide well below those previously used in the treatment of other therapeutic conditions treatable with diazoxide result in an unexpected and advantageous effect by improving clinical manifestations of CNS demyelinating diseases such as multiple sclerosis (MS), acute disseminated encephalomyelitis (ADEM) or post-vaccinal encephalitis (PVE) without causing undesired side effects, such as hyperglycemia, normally present when diazoxide is administered at doses used until now in therapy.

In one aspect, the invention relates to the use of diazoxide or a pharmaceutically acceptable salt thereof for the manufacture of a medicament for the treatment of a mammal afflicted with a CNS demyelinating disease selected from multiple sclerosis (MS), acute disseminated encephalomyelitis (ADEM) and post-vaccinal encephalitis (PVE) wherein the medicament is prepared for the administration of a daily dose of diazoxide of from 0.075 mg/m$^2$/day to 12.00 mg/m$^2$/day.

In another aspect, the invention relates to a pharmaceutical composition comprising from 0.12 mg to 19.5 mg of diazoxide or a pharmaceutically acceptable salt thereof for use in the treatment of a human afflicted with a CNS demyelinating disease selected from multiple sclerosis (MS), acute disseminated encephalomyelitis (ADEM) and post-vaccinal encephalitis (PVE).

In yet another aspect, the invention relates to a method of treatment of a mammal suffering from a CNS demyelinating disease selected from multiple sclerosis (MS), acute disseminated encephalomyelitis (ADEM) and post-vaccinal encephalitis (PVE), comprising the administration of an amount of diazoxide or a pharmaceutically acceptable salt thereof ranging from 0.075 mg/m$^2$/day to 12.00 mg/m$^2$/day.

## BRIEF DESCRIPTION OF THE FIGURES

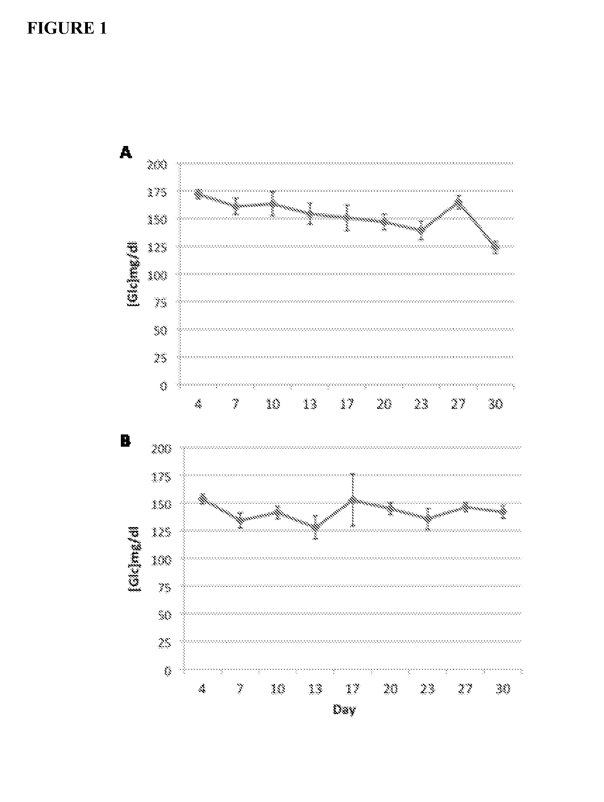

**Figure 1.** Graphics showing the blood glucose level (expressed on mg/dl) 60 minutes after diazoxide administration on mice during one-month treatment. **A:** diazoxide dose 1 mg/kg/day (3 mg/m$^2$/day); **B**: diazoxide dose 0.05 mg/kg/day (0.15 mg/m$^2$/day). Results expressed as mean $\pm$ standard error of the mean (SEM).

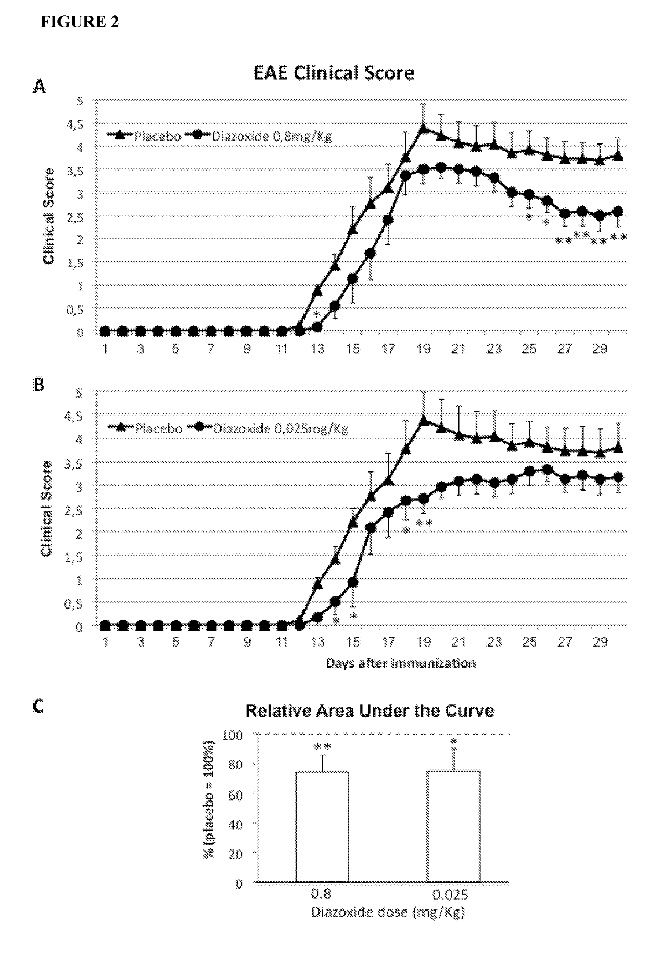

**Figure 2.** Graphic representation of daily EAE clinical score of the animals treated with diazoxide 0.8 mg/kg (2.4 mg/m$^2$/day) (A) or with diazoxide 0.025 mg/kg/day (0.07 mg/m$^2$/day) (B) compared to the placebo-control group. Results expressed as mean $\pm$ SEM. *: p $\leq$ 0.05; **: p $\leq$ 0.01 (non-parametric Mann-Whitney-Wilcoxon test for daily comparison). Graphic representation of global effect of the treatment along all the study is expressed as Relative Area Under the Curve of all diazoxide-treated groups compared to the placebo-control group (=100%) (C). For each group: n $\geq$ 8. Results expressed as mean $\pm$ SEM. *: p $\leq$ 0.05; **: p $\leq$ 0.01 (One-way-Anova for Area Under the Curve comparison).

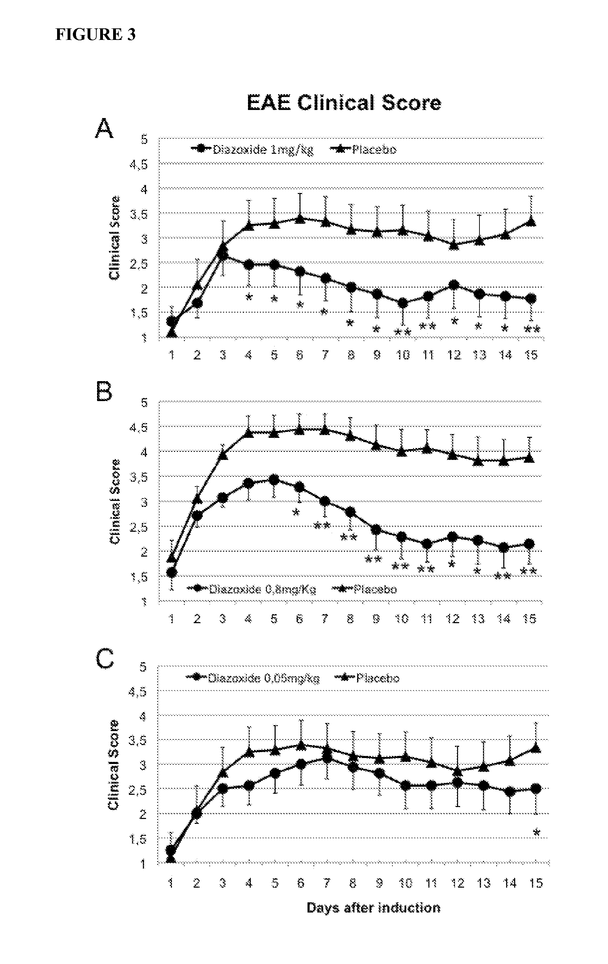

**Figure 3.** Graphic representation of daily EAE clinical score of the animals treated with diazoxide 1 mg/kg/day (3 mg/m$^2$/day) (A), with diazoxide 0.8 mg/kg (2.4 mg/m$^2$/day) (B) or with diazoxide 0.05 mg/kg/day (0.15 mg/m$^2$/day) (C) compared to the placebo-control group. For each group: n $\geq$ 8. Results expressed as mean $\pm$ SEM. *: p $\leq$ 0.05; **: p $\leq$ 0.01 (non-parametric Mann-Whitney-Wilcoxon test for daily comparison).

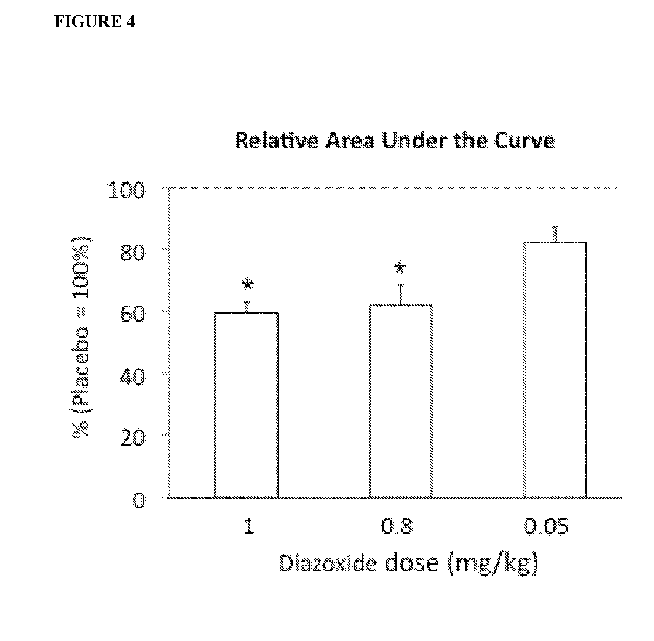

**Figure 4.** Graphic representation of the global effect of the treatment along all the study, expressed as Relative Area Under the Curve of all diazoxide-treated groups compared to the placebo-control group (=100%). For each group: n $\geq$ 8. Results expressed as mean $\pm$ SEM. *: p $\leq$ 0.05; **: p $\leq$ 0.01 (One-way-Anova for Area Under the Curve comparison).

## DETAILED DESCRIPTION OF THE INVENTION

Inventors have surprisingly found that diazoxide is effective in the treatment of a CNS demyelinating disease such as multiple sclerosis (MS), acute disseminated encephalomyelitis (ADEM) and post-vaccinal encephalitis (PVE) in a daily dose which is well below the dose used for the treatment of others conditions such as hypoglycemia in humans, thus avoiding the hyperglycaemic effect, which is undesirable when treating a patient with a demyelinating disease who normally will not suffered from hypoglycemia. This is especially surprising as the prior art suggest that substantially higher doses of diazoxide are necessary to achieve therapeutic effects in the CNS.

It is one aspect of the present invention the use of diazoxide or a pharmaceutically acceptable salt thereof for the manufacture of a medicament for the treatment of a mammal afflicted with a CNS demyelinating disease selected from multiple sclerosis (MS), acute disseminated encephalomyelitis (ADEM) and post-vaccinal encephalitis (PVE) wherein the medicament is prepared for the administration of a daily dose of diazoxide or a pharmaceutically acceptable salt thereof of from 0.075 mg/m$^2$/day to 12.00 mg/m$^2$/day, preferably 0.075 mg/m$^2$/day to 3.70 mg/m$^2$/day, preferably 0.075 mg/m$^2$/day to 3.00 mg/m$^2$/day, preferably 0.075 mg/m$^2$/day to 2.60 mg/m$^2$/day, more preferably 0.075 mg/m$^2$/day to 2.40 mg/m$^2$/day, even more preferably 0.075 mg/m$^2$/day to 1.50 mg/m$^2$/day, and most preferably 0.075 mg/m$^2$/day to 0.75 mg/m$^2$/day. It is further preferred that the medicament is prepared for the administration of a daily dose of diazoxide of from 0.075 mg/m$^2$/day to 0.37 mg/m$^2$/day. The quantities are expressed as amount of diazoxide free base.

In a specific embodiment the medicament is prepared for the administration of a daily dose of diazoxide of 1.85 mg/m$^2$/day.

The term "pharmaceutically acceptable" refers to those properties and/or substances which are acceptable to the patient from a pharmacological/toxicological point of view and to the manufacturing pharmaceutical chemist from a physical/chemical point of view regarding composition, formulation, stability, patient acceptance and bioavailability.

The term "pharmaceutically acceptable salt" embraces salts with a pharmaceutically acceptable acid or base. Pharmaceutically acceptable acids include both inorganic acids, for example and without limitation hydrochloric, sulfuric, phosphoric, diphosphoric, hydrobromic, hydroiodic and nitric acid and organic acids, for example and without limitation citric, fumaric, maleic, malic, mandelic, ascorbic, oxalic, succinic, tartaric, benzoic, acetic, methanesulphonic, ethanesulphonic, benzenesulphonic, cyclohexylsulfamic (cyclamic) or p-toluenesulphonic acid. Pharmaceutically acceptable bases include alkali metal (e.g. sodium or potassium) and alkali earth metal (e.g. calcium or magnesium) hydroxides and organic bases, for example and without limitation alkyl amines, arylalkyl amines and heterocyclic amines.

Oral and parenteral formulations are preferred.

The term "medicament" refers to a pharmaceutical composition comprising diazoxide or a pharmaceutically acceptable salt thereof. The medicament may be administered orally, by injection, by inhalation or insulation or by the rectal route. All these pharmaceutical compositions are prepared by conventional means with pharmaceutically acceptable excipients.

In one embodiment of the present invention the medicament is prepared for oral administration. Pharmaceutical compositions for oral administration are in any suitable dosage form such as syrups, solutions, suspensions, tablets, capsules, lozenges, controlled-release preparations, fast-dissolving preparations or they may be presented as a dry product for reconstitution with water or other suitable vehicle before use

Pharmaceutical compositions for injection (subcutaneous, intradermic, intramuscular, intravenous, etc.) including suspensions, solutions, emulsions in oily or aqueous vehicles, pastes and implantable sustained-release or biodegradable formulations. Such formulations can further comprise one or more additional ingredients including suspending, stabilizing and/or dispersing agents. In one embodiment of a formulation for parenteral administration, the active ingredient is provided in dry (i. e. powder or granular) form for reconstitution with a suitable vehicle (e. g. sterile pyrogen-free water) prior to parenteral administration of the reconstituted composition. Diazoxide may be formulated for parenteral administration by bolus injection or continuous infusion.

Other parentally-administrable formulations which are useful include those which comprise the active ingredient in microcrystalline form, in a liposomal preparation, or as a component of a biodegradable polymer system. Compositions for sustained release or implantation can comprise pharmaceutically acceptable polymeric or hydrophobic materials such as an emulsion, an ion exchange resin, a sparingly soluble polymer, or a sparingly soluble salt.

The pharmaceutical formulations may conveniently be presented in unit dosage form and may be prepared by any of the methods well known in the art of pharmacy. All methods include the step of bringing the active ingredient(s) association with the carrier. In general the formulations are prepared by uniformly and intimately bringing into association the active ingredient with liquid carriers or finely divided solid carriers or both and then, if necessary, shaping the product into the desired formulation.

Formulations of the present invention suitable for oral administration may be presented as discrete units such as capsules, cachets or tablets each containing a predetermined amount of the active ingredient; as a powder or granules; as a solution or a suspension in an aqueous liquid or a non-aqueous liquid; or as an oil-in-water liquid emulsion or a water-in-oil liquid emulsion. The active ingredient may also be presented as a bolus, electuary or paste.

A syrup formulation will generally consist on a suspension or solution of the compound or salt in a liquid carrier for example natural, synthetic or semisynthetic oils or water with flavouring, sweetener and/or colouring agent.

When the composition is in the form of a tablet, any pharmaceutical carrier routinely used for preparing solid formulations may be used.

A tablet may be made by compression or moulding, optionally with one or more accessory ingredients. Compressed tablets may be prepared by compressing in a suitable machine the active ingredient in a free-flowing form such as a powder or granules, optionally mixed with binders, lubricants, inert diluents, surface active or dispersing agents. Moulded tablets may be made by moulding in a suitable machine a mixture of the powdered blend comprising the active compounds moistened with an inert liquid diluent and optionally dried and sieved. The tablets may optionally be coated or scored and may be formulated so as to provide modified (i. e. slow or controlled) release of the active ingredient therein.

Where the composition is in the form of a capsule, any routine encapsulation is suitable, for example using the aforementioned carriers in a hard gelatine capsule. Where the composition is in the form of a soft gelatine capsule any pharmaceutical carrier routinely used for preparing dispersions or suspensions may be considered.

Formulations for injection may be presented in unit dosage form (e.g. in ampoules) or in multidose containers with an added preservative.

The term treatment is used to designate the administration of diazoxide or its pharmaceutical acceptable salt or compositions thereof to control disease progression before or after the clinical signs had appeared. By control of the disease progression it is meant to designate beneficial or desired clinical results including, but not limited to, reduction of symptoms, reduction of the length of the disease, stabilization pathological state (specifically avoidance of further deterioration), delay in the disease's progression, improvement of the pathological state and remission (both partial and total). "Control of disease progression can also entail prolonged survival, compared to the expected survival if the treatment is not applied. In a particular embodiment of the invention diazoxide is used to control of the disease progression once at least one of the disease's clinical signs has appeared.

The term "mammal" includes, but is not restricted to, domestic and farm mammals, primates and humans, e.g., human beings, non-human primates, cows, horses, pigs, sheep, goats, dogs, cats or rodents. In a preferred embodiment the mammal is a human.

A CNS autoimmune demyelinating disease is a disease of the central nervous system in which an autoimmune process participates in the damage of the myelin sheath of neurons. Examples of such diseases are multiple sclerosis (MS), acute disseminated encephalomyelitis (ADEM) and post-vaccinal encephalitis (PVE). Preferably the CNS demyelinating disease is multiple sclerosis.

There is a lack of a natural non human counterpart for MS. Therefore, EAE model is an accepted animal model of MS characterized by demyelination and neurological dysfunction along with corresponding clinical symptoms (Baxter AG. The origin and application of experimental autoimmune encephalomyelitis. Nat Rev Immunol 2007, 7:904-912).

The experiments of the present invention were performed in the experimental autoimmune encephalomyelitis model, sometimes called experimental allergic encephalomyelitis (EAE) model. EAE is an inflammatory demyelinating disease of the CNS that acts as an animal model of brain inflammation. It is mostly used with rodents and is widely studied as an animal model of the human CNS demyelinating diseases, including multiple sclerosis (MS), acute disseminated encephalomyelitis (ADEM) and post-vaccinal encephalitis (PVE). Susceptible rodents, but also primates, develop an autoimmune disease after an injection of CNS myelin homogenate, myelin oligodendrocyte glycoprotein (MOG), myelin basic protein (MBP), proteolipid protein (PLP) or a myelin peptide such as $MOG_{35-55}$. The antigen must be emulsified with adjuvant, such as complete Freund's adjuvant (CFA) with inactivated *Mycobacterium tuberculosis* and *pertussis* toxin, which provide the signals to induce the EAE model. MS has several forms of presentation, including episodic acute period of worsening (the most common, initially), gradual progressive deterioration of neurologic functions or combinations of both. These different presentations have been given standardized names, which are relapsing-remitting (RR), secondary progressive (SP), primary progressive (PP), and progressive relapsing (PR). RR-MS is defined as clearly defined disease relapses with either full recovery or sequelae and residual deficit upon recovery; period between disease relapses are characterized by a lack of disease progression. Approximately 85% of patients start out with RR-MS (Lublin FD and Reingold SC. Defining the clinical course of multiple sclerosis: results of an international survey. National Multiple Sclerosis Society (USA) Advisory Committee on Clinical Trials of New Agents in Multiple Sclerosis. Neurology 1996, 46:907-911; Committee on Multiple Sclerosis. Clinical and biological features. In: Joy JE, Johnston RB Jr, eds. Multiple Sclerosis status and strategies for the future. Washington, DC: National Academies Press 2001:30). In an embodiment of the present invention, the disease to be treated is the relapsing-remitting form of multiple sclerosis (RR-MS).

In an embodiment of the present invention the medicament comprises diazoxide as a sole therapeutic agent. However, it may be advantageous to treat MS with a multiple approach using more than one therapeutic agent useful in the treatment of multiple sclerosis. The beneficial use of diazoxide in the treatment of multiple sclerosis in mammals could also be of high value when combined with another treatment for this disease. Thus, in another embodiment of the present invention the medicament is prepared for the combined administration of diazoxide and one or more therapeutic agents useful in the treatment of multiple sclerosis.

The term "therapeutic agent useful in the treatment of multiple sclerosis" is referred to an agent suitable to be used to treat multiple sclerosis. Therapeutic agents useful in the treatment of multiple sclerosis include, without limitation, fingolimod, laquinimod, teriflunomide, fumaric acid esters, cladribine, mycophenolate mofetil, atorvastatin, interferon-beta, glatiramer acetate, mitoxantrone, cyclophosphamide, natalizumab, daclizumab, rituximab, ustekinumab, ocrelizumab, alemtuzumab, valacyclovir, CTLA4Ig, atacicept.

By "combined administration" it has to be understood that diazoxide can be administered together or separately, simultaneously, concurrently or sequentially with a therapeutic agent useful in the treatment of multiple sclerosis in any order, e.g. the administration of diazoxide can be made first, followed by the administration of one or more therapeutic agent(s) useful in the treatment of multiple sclerosis; or the administration of diazoxide can be made last, preceded by the administration of one or more therapeutic agent(s) useful in the treatment of multiple sclerosis; or the administration of diazoxide can be made concomitantly with one or more therapeutic agent(s) useful in the treatment of multiple sclerosis.

A person skilled in the art understands, in the context of the present invention, that the medicament for combined administration of diazoxide and an additional therapeutic agent useful in the treatment of multiple sclerosis can be in the form of a single dosage form or in separate dosage forms. In an embodiment of the present invention, the medicament comprises diazoxide and one or more additional therapeutic agent(s) useful in the treatment of multiple sclerosis selected from fingolimod, laquinimod, teriflunomide, fumaric acid esters, cladribine, mycophenolate mofetil, atorvastatin, interferon-beta, glatiramer acetate, mitoxantrone, cyclophosphamide, natalizumab, daclizumab, rituximab, ustekinumab, ocrelizumab, alemtuzumab, valacyclovir, CTLA4Ig and atacicept.

In another embodiment of the present invention diazoxide and the additional therapeutic agent are contained in a single dosage form. Additional therapeutic agents suitable for combination with diazoxide in a single dosage form are fingolimod, laquinimod, teriflunomide, acid fumaric esters, cladribine, mycophenolate mofetil and atorvastatin, preferably fingolimod, laquinimod, teriflunomide and acid fumaric esters, more preferably fingolimod, laquinimod and teriflunomide, most preferably fingolimod and laquinimod.

In another embodiment of the present invention diazoxide and the additional therapeutic agent are contained in separate dosage forms. Additional therapeutic agents suitable for combined treatment with diazoxide in a separate dosage forms are interferon beta, glatiramer acetate, mitoxantrone, cyclophosphamide, natalizumab, fingolimod, laquinimod, fumaric acid esters, teriflunomide, cladribine, mycophenolate mofetil, daclizumab, rituximab, ustekinumab, ocrelizumab, alemtuzumab, valacyclovir, CTLA4Ig, atorvastatin and atacicept; preferably interferon beta, glatiramer acetate, mitoxantrone, cyclophosphamide and natalizumab; more preferably interferon beta, glatiramer acetate and mitoxantrone; most preferably interferon beta.

According to the present invention, diazoxide is found to be effective for the treatment of MS at a dose below 12 mg/m2/day (0,324 mg/kg/day in humans) which is much lower than the doses currently used for hypoglycemia and hypertensive treatments (3-8 mg/kg/day and 150-600 mg/kg/day, respectively).

When diazoxide is used for the treatment of a human patient it will be administered in quantities ranging from 0.002 mg/kg/day to 0.324 mg/kg/day, preferably 0.002 mg/kg/day to 0,1 mg/kg/day, preferably 0.002 mg/kg/day to 0.081 mg/kg/day, preferably 0.002 mg/kg/day to 0.07 mg/kg/day, more preferably 0.002 mg/kg/day to 0.065 mg/kg/day, even more preferably 0.002 mg/kg/day to 0.041 mg/kg/day, most preferably 0.002 mg/kg/day to 0.020 mg/kg/day and still most preferably from 0.002 mg/kg/day to 0.010 mg/kg/day. These doses, in the case of a patient having an average body weight of 60 Kg, correspond to daily doses ranging from 0.12 mg/day to 19.5 mg/day, preferably 0.12 mg/day to 6 mg/day, preferably 0.12 mg/day to 4.86 mg/day, preferably 0.12 mg/day to 4.22 mg/day, more preferably 0.12 mg/day to 3.89 mg/day, even more preferably 0.12 mg/day to 2.43 mg/day, most preferably 0.12 mg/day to 1.22 mg/day and still most preferably from 0.12 mg/day to 0.60 mg/day. The quantities are expressed as amount of diazoxide free base.

These daily doses can be administered once a day or divided into two or three equal doses administered along the day. The diazoxide content in the pharmaceutical composition will vary according to the number of daily administrations.

Thus according to another aspect the present invention provides a unit dose composition formulated for administration three times a day will contain from 0.04 mg to 6.50 mg, preferably 0.04 mg to 2 mg, preferably 0.04 mg to 1.62 mg, preferably 0.04 mg to 1.41 mg, more preferably 0.04 mg to 1.30 mg, even more preferably 0.04 mg to 0.81 mg, most preferably 0.04 mg to 0.41 mg and still most preferably from 0.04 mg to 0.20 mg of diazoxide or a pharmaceutically acceptable salt thereof for use in the treatment of a human afflicted with a CNS demyelinating disease selected from multiple sclerosis (MS), acute disseminated encephalomyelitis (ADEM) and post-vaccinal encephalitis (PVE).

According to another aspect the present invention provides a unit dose composition formulated for administration twice a day will contain from 0.06 mg to 9.75 mg, preferably 0.06 mg to 3 mg, preferably 0.06 mg to 2.43 mg, preferably 0.06 mg to 2.11 mg, more preferably 0.06 mg to 1.95 mg, even more preferably 0.06 mg to 1.21 mg, most preferably 0.06 mg to 0.61 mg and still most preferably from 0.06 mg to 0.30 mg of diazoxide or a pharmaceutically acceptable salt thereof for use in the treatment of a human afflicted with a CNS demyelinating disease selected from multiple sclerosis (MS), acute disseminated encephalomyelitis (ADEM) and post-vaccinal encephalitis (PVE).

According to still another aspect the present invention provides a unit dose composition formulated for administration once a day will contain from 0.12 mg to 19.5 mg, preferably 0.12 mg to 6 mg, preferably 0.12 mg to 4.86 mg, preferably 0.12 mg to 4.22 mg, more preferably 0.12 mg to 3.89 mg, even more preferably 0.12 mg to 2.43 mg, most preferably 0.12 mg to 1.22 mg and still most preferably from 0.12 mg to 0.60 mg of diazoxide or a pharmaceutically acceptable salt thereof for use in the treatment of a human afflicted with a CNS demyelinating disease selected from multiple sclerosis (MS), acute disseminated encephalomyelitis (ADEM) and post-vaccinal encephalitis (PVE).

In a particular embodiment the present invention provides a unit dose composition comprising diazoxide or a pharmaceutically acceptable salt thereof in an amount selected from 1.0 mg, 1.5 mg and 3.0 mg for use in the treatment of a human afflicted with a CNS demyelinating disease selected from multiple sclerosis (MS), acute disseminated encephalomyelitis (ADEM) and post-vaccinal encephalitis (PVE).

In the present invention, the term "pharmaceutical composition" is equivalent to the term "medicament" as used herein.

The pharmaceutical composition can be an oral dosage form intended for once a day or twice a day administration. This facilitates adhesion of the patient to the therapeutic regime and thus compliance with this regime.

In another aspect, the invention is directed to a method of treatment of a mammal suffering from a CNS demyelinating disease selected from multiple sclerosis (MS), acute disseminated encephalomyelitis (ADEM) and post-vaccinal encephalitis (PVE), comprising the administration of an amount of diazoxide or a pharmaceutically acceptable salt thereof ranging from 0.075 mg/m$^2$/day to 3.00 mg/m$^2$/day, preferably 0.075 mg/m$^2$/day to 2.60 mg/m$^2$/day, more preferably 0.075 mg/m$^2$/day to 2.40 mg/m$^2$/day, more preferably 0.075 mg/m$^2$/day to 1.90 mg/m$^2$/day, even more preferably 0.075 mg/m$^2$/day to 1.50 mg/m$^2$/day, and most preferably 0.075 mg/m$^2$/day to 0.75 mg/m$^2$/day. It is further preferred to administer an amount of diazoxide or a pharmaceutically acceptable salt thereof ranging from 0.075 mg/m$^2$/day to 0.37 mg/m$^2$/day.

When diazoxide is used for the treatment of a human patient the method of treatment comprises the administration of an amount of diazoxide or a pharmaceutically acceptable salt thereof ranging from 0.002 mg/kg/day to 0.324 mg/kg/day, preferably 0.002 mg/kg/day to 0,1 mg/kg/day, preferably 0.002 mg/kg/day to 0.081 mg/kg/day, preferably 0.002 mg/kg/day to 0.07 mg/kg/day, more preferably 0.002 mg/kg/day to 0.065 mg/kg/day, even more preferably 0.002 mg/kg/day to 0.041 mg/kg/day, most preferably 0.002 mg/kg/day to 0.020 mg/kg/day and still most preferably from 0.002 mg/kg/day to 0.010 mg/kg/day. These doses, in the case of a patient having an average body weight of 60 Kg, correspond to daily doses ranging from 0.12 mg/day to 19.5 mg/day, preferably 0.12 mg/day to 6 mg/day, preferably 0.12 mg/day to 4.86 mg/day, preferably 0.12 mg/day to 4.22 mg/day, more preferably 0.12 mg/day to 3.89 mg/day, even more preferably 0.12 mg/day to 2.43 mg/day, most preferably 0.12 mg/day to 1.22 mg/day and still most preferably from 0.12 mg/day to 0.60 mg/day. The quantities are expressed as amount of diazoxide free base.

These daily doses can be administered once a day or divided into two or three equal doses administered along the day. The amount of diazoxide administered will vary according to the number of daily administrations.

Thus according to another aspect the present invention provides a method of treatment comprising the administration three times a day of an amount of diazoxide or a pharmaceutically acceptable salt thereof ranging from 0.04 mg to 6.50 mg, preferably 0.04 mg to 2 mg, preferably 0.04 mg to 1.62 mg, preferably 0.04 mg to 1.41 mg, more preferably 0.04 mg to 1.30 mg, even more preferably 0.04 mg to 0.81 mg, most preferably 0.04 mg to 0.41 mg and still most preferably from 0.04 mg to 0.20 mg.

In yet another aspect the present invention provides a pharmaceutical composition comprising from 0.04 mg to 6.50 mg, preferably 0.04 mg to 2 mg, preferably 0.04 mg to 1.62 mg, preferably 0.04 mg to 1.41 mg, more preferably 0.04 mg to 1.30 mg, even more preferably 0.04 mg to 0.81 mg, most preferably 0.04 mg to 0.41 mg and still most preferably from 0.04 mg to 0.20 mg of diazoxide or a pharmaceutically acceptable salt thereof for administration three times a day in the treatment of multiple sclerosis (MS), acute disseminated encephalomyelitis (ADEM) and post-vaccinal encephalitis (PVE).

According to another aspect the present invention provides a method of treatment comprising the administration twice a day of an amount of diazoxide or a pharmaceutically acceptable salt thereof ranging from 0.06 mg to 9.75 mg, preferably 0.06 mg to 3 mg, preferably 0.06 mg to 2.43 mg, preferably 0.06 mg to 2.11 mg, more preferably 0.06 mg to 1.95 mg, even more preferably 0.06 mg to 1.21 mg, most preferably 0.06 mg to 0.61 mg and still most preferably from 0.06 mg to 0.30 mg.

In yet another aspect the present invention provides a pharmaceutical composition comprising from 0.06 mg to 9.75 mg, preferably 0.06 mg to 3 mg, preferably 0.06 mg to 2.43 mg, preferably 0.06 mg to 2.11 mg, more preferably 0.06 mg to 1.95 mg, even more preferably 0.06 mg to 1.21 mg, most preferably 0.06 mg to 0.61 mg and still most preferably from 0.06 mg to 0.30 mg of diazoxide or a pharmaceutically acceptable salt thereof for administration twice a day in the treatment of multiple sclerosis (MS), acute disseminated encephalomyelitis (ADEM) and post-vaccinal encephalitis (PVE).

According to still another aspect the present invention provides a method of treatment comprising the administration once a day of an amount of diazoxide or a pharmaceutically acceptable salt thereof ranging from 0.12 mg to 19.5 mg, preferably 0.12 mg to 6 mg, preferably 0.12 mg to 4.86 mg, preferably 0.12 mg to 4.22 mg, more preferably 0.12 mg to 3.89 mg, even more preferably 0.12 mg to 2.43 mg, most preferably 0.12 mg to 1.22 mg and still most preferably from 0.12 mg to 0.60 mg.

In yet another aspect the present invention provides a pharmaceutical composition comprising from 0.12 mg to 19.5 mg, preferably 0.12 mg to 6 mg, preferably 0.12 mg to 4.86 mg, preferably 0.12 mg to 4.22 mg, more preferably 0.12 mg to 3.89 mg, even more preferably 0.12 mg to 2.43 mg, most preferably 0.12 mg to 1.22 mg and still most preferably from 0.12 mg to 0.60 mg of diazoxide or a pharmaceutically acceptable salt thereof for administration once a day in the treatment of multiple sclerosis (MS), acute disseminated encephalomyelitis (ADEM) and post-vaccinal encephalitis (PVE).

In particular embodiments diazoxide or a pharmaceutically acceptable salt thereof is administered in an amount selected from 1.85 mg/m$^2$/day, 3.7 mg/m$^2$/day and 11.1 mg/m$^2$/day.

In one embodiment the method is characterized in that diazoxide or a pharmaceutically acceptable salt thereof is administered orally.

In one embodiment of the present invention the method is characterized in that diazoxide or a pharmaceutically acceptable salt thereof is administered as a sole therapeutic agent. In other embodiment the method comprises the administration of an amount of diazoxide or a pharmaceutically acceptable salt thereof in combination with one or more therapeutic agents useful in the treatment of multiple sclerosis. In a preferred embodiment the diazoxide and the additional therapeutic agent are contained in a single dosage form. In other preferred embodiment diazoxide and the additional therapeutic agent are contained in separate dosage forms.

In one embodiment of the present invention the method is characterized in that diazoxide or a pharmaceutically acceptable salt thereof is administered as a dosage form intended for once a day administration. In another embodiment it is intended for twice a day administration.

In a preferred embodiment of the present invention the method is used to treat humans.

In an embodiment of the present invention the method of treatment is used to treat multiple sclerosis including primary progressive MS (PP-MS), secondary progressive MS (SP-MS), progressive-relapsing MS (PR_MS) and the relapsing-remitting form of multiple sclerosis form (RR-MS), more preferably the relapsing-remitting multiple sclerosis form (RR-MS).

## EXAMPLES

Experiments in the present invention, performed in mice, had shown that diazoxide is effective at different daily doses below 12 $mg/m^2$/day.

Doses of diazoxide may be expressed either in mg of diazoxide per kg of body weight or in mg of diazoxide per square meter of body surface. The article from Reagan-Shaw S. "Dose translation from animal to human studies revisited". FASEB J 2007, 22:659-661 provides the standard conversion factors used to convert mg/kg to $mg/m^2$.

$$\text{Dose (mg/kg)} \times K_m = \text{Dose (mg/m}^2\text{)}$$

The article also explains that this conversion is the basis for converting dose in a first animal species to dose in a second animal species (allometric dose translation). Thus, animal dose (AD) in mg/kg can be converted to human equivalent dose (HED) in mg/kg using the following formula:

$$\text{HED (mg/kg)} = \text{AD (mg/kg)} \times \frac{\text{Animal } K_m}{\text{Human } K_m}$$

wherein the $K_m$ for each species is shown in Table I (data extracted from Reagan-Shaw S. Dose translation from animal to human studies revisited. FASEB J 2007, 22:659-661).

**Table I. $K_m$ factor for conversion of AD to HED**

| Species | | $K_m$ factor |
|---|---|---|
| Human | Adult | 37 |
| | Child | 25 |
| Baboon | | 20 |
| Dog | | 20 |
| Monkey | | 12 |
| Rabbit | | 12 |
| Guinea p | | 8 |
| Rat | | 6 |
| Hamster | | 5 |
| Mouse | | 3 |

Thus, the experiments with doses of 0.025 mg/kg/day, 0.05 mg/kg/day, 0.25 mg/kg/day, 0.8 mg/kg/day, 1 mg/kg/day and 10 mg/kg/day in mice correspond to general doses in mammals of 0.075 $mg/m^2$/day, 0.15 $mg/m^2$/day, 0.75 $mg/m^2$/day, 2.4 $mg/m^2$/day, 3 $mg/m^2$/day and 30 $mg/m^2$/day.

The advantages of the invention are more fully illustrated with reference to the following examples.

## EXAMPLE 1: LOW DOSES OF DIAZOXIDE DO NOT CAUSE HYPERGLYCEMIA IN MICE

To determine the *in vivo* effects of very low doses of diazoxide on glycemia, mice receiving a daily administration of diazoxide were monitorized. Female C57BL/6J mice, 11 weeks of age, were purchased from Charles River and maintained on a 12:12 hr light:dark cycle, with standard chow and water freely available. The experiments began at 11:00 h. Diazoxide (Sigma-Aldrich, St. Louis, Mo, USA) was administered daily orally by gavage (p.o.) at doses 1 mg/kg (3 $mg/m^2$) (Figure 1A) and 0.05 mg/kg (0,15 $mg/m^2$) (Figure 1B) (n = 6/group) for an initial period of 4 days. This period of time was considered necessary to create a steady state in plasma diazoxide concentration. From day 4 on, glucose blood levels were measured every 3-4 days during the 30-day treatment period. Measurements were performed immediately before drug administration (time 0) and at 60 minutes. Blood samples were obtained from the saphenous vein and glucose levels were determined using a glucometer and glucose test strips (Accu-Chek® Aviva, Roche Diagnostics, Indianapolis, Ind, USA). For each group, hyperglycemia was considered when glucose concentration was equal or higher than 176 mg/dl. As shown in FIGURE 1 none of the two doses tested in this study produced hyperglycemia during the 30 days period of diazoxide treatment.

## EXAMPLE 2: LOW DOSES OF DIAZOXIDE IMPROVE EXPERIMENTAL AUTOIMMUNE ENCEPHALOMYELITIS IN MICE

Female C57BL/6J mice, 8-10 weeks of age, were purchased from Harlan Laboratories and maintained on a 12:12 hr light:dark cycle, with standard chow and water freely available. EAE was induced by immunization with >95% pure synthetic myelin oligodendrocyte glycoprotein peptide 35-55 (rat $MOG_{35-55}$, sequence: MEVGWYRSPFSRVVH-LYRNGK; EspiKem Srl, Florence, Italy). Mice were injected subcutaneously at one side of the flanks with 100 $\mu$l of a solution containing 150$\mu$g of rat MOG in complete Freund's adjuvant (Sigma-Aldrich, St Louis, Mo, USA) and 5mg/ml *Mycobacterium tuberculosis* H37 RA (Difco Laboratories, Detroit, MI, USA). Mice also received one intraperitoneal injection of 150 ng pertussis toxin (Sigma-Aldrich, St. Louis, Mo, USA) in 100 $\mu$l PBS, immediately after MOG injection, and 48h later.

Mice were scored daily for signs of EAE on a scale 0-6 using the following criteria: 0, no clinical signs; 1: distal limp tail; 1.5: complete limp tail; 2: mild paraparesis of the hind limbs, unsteady gait, impairment of righting reflex; 3: moderate paraparesis, partial hind limb paralysis, voluntary movements still possible, ataxia; 4: paraplegia and forelimb weakness; 5: tetraparesis; 6: moribund state. When clinical signs were intermediate between two grades of disease, 0.5 was added to the lower score (Suen et al. A critical role for lymphotoxin in experimental allergic encephalomyelitis. J Exp Med 1997, 186:1233-40).

The same day the mice were immunized, they were randomly assigned to a diazoxide-treatment or control group. The period of treatment was 30 days, starting at the point of immunization, and the drug was dissolved in a vehicle consisting in 98,5% water plus 1,5% of dimetyl sulfoxide (Sigma-Aldrich, St. Louis, Mo, USA) and administered daily orally by gavage (p.o.), in a dose volume of 200 $\mu$l. Diazoxide (Sigma-Aldrich, St. Louis, Mo, USA) was administered at doses of 0.025 mg/kg (0.075 $mg/m^2$), and 0.8 mg/kg (2.4 $mg/m^2$). One control group received a daily oral administration of vehicle.

The non-parametric Mann-Whitney-Wilcoxon test was performed to establish the differences between the different treated groups for each day. $p < 0.05$ was considered as significant. To test the global effect of the treatment along all the study, the Area Under the Curve of treated groups and control group was compared using One-Way-Anova test. $p < 0.05$ was considered as significant.

From 9 days post-EAE immunization, some mice began to display less exploratory activity and less feeding behaviour as well as a loss of body weight. At day 12, several mice showed the signs of onset, including flaccid tail and hind leg paresis. These symptoms gradually aggravated, leading to complete paralysis, tetraplegia even morbidity. Clinical signs reached the peak around day 15, lasted for additional 4-6 days and then followed by a gradual slight recovery (FIGURE 2).

All diazoxide dosages tested showed a better mean value for clinical score when compared to the control group after day 13 post-immunization. For the dosages 0.8 mg/kg clinical values were significantly lower at day thirteen and from day twenty-five to thirty when compared to the placebo control group (FIGURE 2A). For the 0.025 mg/kg (0.075 $mg/m^2$) diazoxide-treated group there was a significant difference at days fourteen, fifteen, eighteen and nineteen when compared to the control group (FIGURE 2B).

Starting on the first day of treatment, mice receiving both doses of diazoxide presented a global mean of 26% significant decrease (p<0.05 for the 0,025 mg/Kg (0.075 $mg/m^2$) dose and p<0,01 for the 0,8 mg/Kg (2.4 $mg/m^2$) dose)

of the clinical score (FIGURE 2C).

**EXAMPLE 3: TREATMENT WITH LOW DOSES OF DIAZOXIDE AFTER THE FIRST CLINICAL SIGNS IMPROVE EXPERIMENTAL AUTOIMMUNE ENCEPHALOMYELITIS IN MICE**

Experimental Autoimmune Encephalomyelitis induction and clinic scoring of mice were performed as described on Example 2.

The day scoring a value of 1, every mouse was randomly assigned to a diazoxide-treatment or placebo-control group. The period of treatment was 15 days and the drug was disolved in a vehicle consisting in 98,5% water plus 1,5% of dimetyl sulfoxide (Sigma-Aldrich, St. Louis, Mo, USA) and was administered daily orally by gavage (p.o.), in a dose volume of 200$\mu$l Diazoxide (Sigma-Aldrich, St. Louis, Mo, USA) was administered at doses of 0.05 mg/kg (0.15 mg/m$^2$), 0.8 mg/kg (2.4 mg/m$^2$) and 1 mg/kg (3 mg/m$^2$). One placebo-control group received a daily oral administration of vehicle.

The non-parametric Mann-Whitney-Wilcoxon test was performed to establish the differences between the treated groups and control group for each day. $p < 0.05$ was considered as significant. To test the global effect of the treatment along all the study, the Area Under the Curve of treated groups and control group was compared using One-Way-Anova test. $p < 0.05$ was considered as significant.

Compared to the placebo-control group, the treatment with diazoxide evidenced a significant reduction of morbidity and of the mean daily clinical score of mice at the three doses tested (0.05 mg/kg (0.15 mg/m$^2$), 0.8 mg/kg (2.4 mg/m$^2$) and 1 mg/kg (3 mg/m$^2$)) (FIGURES 3 and 4).

For dosages 1mg/kg (3 mg/m$^2$) and 0.8 mg/kg (2.4 mg/m$^2$) all clinical values were significantly lower from day four (FIGURE 3A) and six respectively (FIGURE 3B) to fifteen when compared to the placebo-control group. For the 0.05 mg/kg (0.15 mg/m$^2$) diazoxide-treated group there was a significant difference at the final phase of the study when compared to the control group (FIGURE 3C).

Starting on the first day of treatment, mice receiving a dose of 0.05 mg/kg (0.15 mg/m$^2$) presented a global mean of 19% decrease of the clinical score. This reduction significantly reached 40% in mice receiving daily doses of 0.8 mg/kg (2.4 mg/m$^2$) and 1 mg/kg (3 mg/m$^2$) (p<0.05) (FIGURE 4).

Taken together, results from examples 1, 2 and 3 clearly demonstrate that a standard pharmacological preparation of a low-dose of diazoxide reduces clinical signs of multiple sclerosis in EAE mice, with no effects on glycemia. Thus, low doses of diazoxide can be included in a pharmaceutical composition suitable for treatment of a mammal afflicted with a CNS demyelinating disease selected from multiple sclerosis (MS), acute disseminated encephalomyelitis (ADEM) and post-vaccinal encephalitis (PVE).

**Claims**

1. Use of diazoxide or a pharmaceutically acceptable salt thereof for the manufacture of a medicament for the treatment of a mammal afflicted with an autoimmune CNS demyelinating disease wherein the medicament is prepared for the administration of a daily dose of diazoxide or of a pharmaceutically acceptable salt thereof of from 0.075 mg/m$^2$/day to 12.00 mg/m$^2$/day expressed as mg/m$^2$/day of diazoxide free base.

2. Use according to claim 1 wherein diazoxide is used.

3. Use according to any preceding claim, wherein the mammal is a human.

4. Use according to any preceding claim, wherein the disease is selected from multiple sclerosis (MS), acute disseminated encephalomyelitis (ADEM) and post-vaccinal encephalitis (PVE).

5. Use according to claim 4, wherein the disease is multiple sclerosis.

6. Use according to any preceding claim, wherein the medicament is prepared for oral administration.

7. Use according to any preceding claim, wherein the medicament is prepared for the administration of a daily dose of diazoxide of from 0.075 mg/m$^2$/day to 2.60 mg/m$^2$/day.

8. Use according to claim 7, wherein the medicament is prepared for the administration of a daily dose of diazoxide selected from 0.075 mg/m$^2$/day, 0.15 mg/m$^2$/day, 0.75 mg/m$^2$/day, 1.85 mg/m$^2$/day, 2.22 mg/m$^2$/day, 2.96 mg/m$^2$/day and 3.7 mg/m$^2$/day.

9. Use according to any preceding claim, wherein the medicament comprises diazoxide as sole therapeutic agent.

10. Use according to claims 1 to 9, wherein the medicament is prepared for the combined administration of diazoxide and one or more therapeutic agents useful in the treatment of multiple sclerosis.

11. Use according to claim 10, wherein the medicament comprises diazoxide and one or more additional therapeutic agents useful in the treatment of multiple sclerosis selected from fingolimod, laquinimod, teriflunomide, fumaric acid esters, cladribine, mycophenolate mofetil, atorvastatin, interferon-beta, glatiramer acetate, mitoxantrone, cyclophosphamide, natalizumab, daclizumab, rituximab, ustekinumab, ocrelizumab, alemtuzumab, valacyclovir, CTLA4Ig and atacicept.

12. Use according to claim 10 wherein diazoxide and the additional therapeutic agent are contained in a single dosage form.

13. Use according to claim 10 wherein diazoxide and the additional therapeutic agent are contained in separate dosage forms.

14. Pharmaceutical composition comprising from 0.12 mg to 19.5 mg of diazoxide or a pharmaceutically acceptable salt thereof for use in the treatment of a human afflicted with a CNS demyelinating disease selected from multiple sclerosis (MS), acute disseminated encephalomyelitis (ADEM) and post-vaccinal encephalitis (PVE).

15. Pharmaceutical composition according to claim 14 comprising from 0.12 mg to 4.22 mg of diazoxide or a pharmaceutically acceptable salt thereof for use in the treatment of a human afflicted with a CNS demyelinating disease selected from multiple sclerosis (MS), acute disseminated encephalomyelitis (ADEM) and post-vaccinal encephalitis (PVE).

16. Pharmaceutical composition according to claim 14 comprising an amount of diazoxide or a pharmaceutically acceptable salt thereof selected from 1.0 mg, 1.5 mg and 3.0 mg for use in the treatment of a human afflicted with a CNS demyelinating disease selected from multiple sclerosis (MS), acute disseminated encephalomyelitis (ADEM) and post-vaccinal encephalitis (PVE).

**FIGURE 1**

FIGURE 2

**EAE Clinical Score**

A

B

C

**Relative Area Under the Curve**

**FIGURE 3**

EAE Clinical Score

**FIGURE 4**

Relative Area Under the Curve

Europäisches
Patentamt

European
Patent Office

Office européen
des brevets

## EUROPEAN SEARCH REPORT

Application Number

EP 10 15 0027

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | EP 1 782 812 A1 (NEUROTEC PHARMA S L [ES]) 9 May 2007 (2007-05-09) | 1-7,9 | INV. A61K31/549 A61P25/00 |
| Y | * paragraph [0021] * <br> * claim 1 * <br> * claim 8 * <br> * claim 17 * | 8,10-16 | |
| Y | GRILL V ET AL: "Beneficial effects of K-ATP channel openers in diabetes: an update on mechanisms and clinical experiences." <br> DIABETES, OBESITY & METABOLISM NOV 2009, vol. 11 Suppl 4, November 2009 (2009-11), pages 143-148, XP002575637 <br> ISSN: 1463-1326 <br> * page 145, column 2, paragraph 2 * | 8,10-16 | |
| Y | RAM C V S ET AL: "Individual titration of diazoxide dosage in the treatment of severe hypertension" <br> AMERICAN JOURNAL OF CARDIOLOGY, CAHNERS PUBLISHING CO., NEWTON, MA, US, <br> vol. 43, no. 3, 1 March 1979 (1979-03-01), pages 627-630, XP023195164 <br> ISSN: 0002-9149 <br> [retrieved on 1979-03-01] <br> * abstract * <br> * page 628; figure 3 * <br> * page 629, paragraph 4 * <br> * page 629; table 2 * | 8,10-16 | TECHNICAL FIELDS SEARCHED (IPC) <br><br> A61K |

The present search report has been drawn up for all claims

| Place of search | Date of completion of the search | Examiner |
|---|---|---|
| Munich | 26 April 2010 | Uryga-Polowy, V |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

  ...........................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 10 15 0027

This annex lists the patent family members relating to the patent documents cited in the above-mentioned European search report.
The members are as contained in the European Patent Office EDP file on
The European Patent Office is in no way liable for these particulars which are merely given for the purpose of information.

26-04-2010

| Patent document cited in search report | | Publication date | Patent family member(s) | | Publication date |
|---|---|---|---|---|---|
| EP 1782812 | A1 | 09-05-2007 | AU | 2005256675 A1 | 05-01-2006 |
| | | | CA | 2571717 A1 | 05-01-2006 |
| | | | WO | 2006000607 A1 | 05-01-2006 |
| | | | JP | 2008503548 T | 07-02-2008 |
| | | | US | 2007254871 A1 | 01-11-2007 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2006000607 A1

### Non-patent literature cited in the description

- **NOSEWORTHY JH et al.** *Multiple sclerosis. N Engl J Med,* 2000, vol. 343, 938-52
- **KURTZKE JF.** Multiple sclerosis: changing time. *Neuroepidemiology,* 1991, vol. 10, 1-8
- **COMPSTON A ; COLES A.** Multiple Sclerosis. *Lancet,* 2008, vol. 372, 1502-17
- **MARTIN R et al.** Immunological aspects of demyelinating diseases. *Ann Rev Immunol,* 1992, vol. 10, 153-187
- **KREUTZBERG GW.** Microglia: a sensor for pathological events in the CNS. *Trends Neurosci,* 1996, vol. 19, 312-3188
- **HEPPNER FL et al.** Experimental autoimmune encephalomyelitis repressed by microglial paralysis. *Nat Med,* 2005, vol. 11, 146-152
- **LUCCHINETTI CF et al.** Multiple sclerosis: lessons from neuropathology. *Semin Neurol.,* 1998, vol. 18, 337-349
- **GOODIN DS.** Interferon-beta-therapy in multiple sclerosis: evidence for a clinically relevant dose response. *Drugs,* 2001, vol. 61, 1693-1703
- **NEUHAUS O et al.** Mechanisms of action of glatiramer acetate in multiple sclerosis. *Neurology,* 2001, vol. 56, 702-708
- **YEDNOCK TA et al.** Prevention of experimental autoimmune encephalomyelitis by antibodies against $\alpha4\beta1$ integrin. *Nature,* 1992, vol. 356, 63-66
- **GOODIN DS et al.** The use of mitoxantrone (Novantrone) for the treatment of multiple sclerosis. Report of the Therapeutics and Technology Assessment subcommittee of the American Academy of Neurology. *Neurology,* 2003, vol. 61, 1332-1338
- **FRANCIS GS et al.** Interferon-beta1-a in MS: results following development of neutralizing antibodies in PRISMS. *Neurology,* 2005, vol. 65, 48-55
- **HARTUNG HP et al.** Mitoxantrone in progressive multiple sclerosis: a placebo-controlled, double-blind, randomised multicentre trial. *Lancet,* 2002, vol. 360, 2018-2025
- **RUDICK RA et al.** Natalizumab plus interferon beta-1a for relapsing multiple sclerosis. *N Engl J Med,* 2006, vol. 354, 911-923
- **RAMONET D et al.** Putative glucosensing property in rat and human activated microglia. *Neurobiol Dis,* 2004, vol. 17, 1-9
- **MANNHOLD R.** KATP channel openers: structure-activity relationships and therapeutic potential. *Med Res Rev,* 2004, vol. 24, 213-66
- **FANG P ; MACDONALD I ; LAVER M ; HUA A ; KINCAID-SMITH P.** Oral diazoxide in uncontrolled malignant hypertension. *Med J Aust.,* 26 October 1974, vol. 2 (17), 621-4
- **GANTENBEIN M et al.** *Life Sciences,* 1995, vol. 57, 113-116
- **FARKAS E et al.** *Brain Research.,* 2004, vol. 1008, 252-260
- **LENZSER G et al.** *Brain Research.,* 2005, vol. 1051, 72-80
- **BAXTER AG.** The origin and application of experimental autoimmune encephalomyelitis. *Nat Rev Immunol,* 2007, vol. 7, 904-912
- **LUBLIN FD ; REINGOLD SC.** Defining the clinical course of multiple sclerosis: results of an international survey. National Multiple Sclerosis Society (USA) Advisory Committee on Clinical Trials of New Agents in Multiple Sclerosis. *Neurology,* 1996, vol. 46, 907-911
- Committee on Multiple Sclerosis. Clinical and biological features. Multiple Sclerosis status and strategies for the future. National Academies Press, 2001, 30
- **REAGAN-SHAW S.** Dose translation from animal to human studies revisited. *FASEB J,* 2007, vol. 22, 659-661
- **SUEN et al.** A critical role for lymphotoxin in experimental allergic encephalomyelitis. *J Exp Med,* 1997, vol. 186, 1233-40